(19) Europäisches Patentamt
European Patent Office
Office européen des brevets

(11) Veröffentlichungsnummer: **0 271 710 B1**

(12) **EUROPÄISCHE PATENTSCHRIFT**

(45) Veröffentlichungstag der Patentschrift: **21.08.91**

(51) Int. Cl.⁵: **H05G 1/06, A61B 6/14**

(21) Anmeldenummer: **87116530.4**

(22) Anmeldetag: **09.11.87**

(54) **Röntgenstrahler, insbesondere zur Anfertigung von intraoralen Zahnaufnahmen.**

(30) Priorität: **21.11.86 DE 3639773**

(43) Veröffentlichungstag der Anmeldung:
**22.06.88 Patentblatt 88/25**

(45) Bekanntmachung des Hinweises auf die
Patenterteilung:
**21.08.91 Patentblatt 91/34**

(84) Benannte Vertragsstaaten:
**DE FR IT SE**

(56) Entgegenhaltungen:
DE-C- 629 610
US-A- 2 137 122
US-A- 3 812 366
US-A- 4 157 476

PATENT ABSTRACTS OF JAPAN, Band 5, Nr.
82 (E-59)[754], 29. Mai 1981 & JP-A-56-30 296

(73) Patentinhaber: **Siemens Aktiengesellschaft**
**Wittelsbacherplatz 2**
**W-8000 München 2(DE)**

(72) Erfinder: **Eitner, Dorothea**
**Würzburger Ring 35**
**W-8520 Erlangen(DE)**
Erfinder: **Kühnke, Hermann**
**Hirtenberg 15**
**W-8521 Münchaurach(DE)**
Erfinder: **Messingschlager, Elisabeth**
**Engelhardsberg 16**
**W-8551 Wiesenttal(DE)**
Erfinder: **Wilke, Dorothea**
**Heidackerstrasse 16**
**W-8520 Erlangen(DE)**

Rank Xerox (UK) Business Services

**Beschreibung**

Die Erfindung betrifft einen Röntgenstrahler, insbesondere zur Anfertigung von intraoralen Zahnaufnahmen, der eine Röntgenröhre und einen Hochspannungsgenerator mit einem Hochspannungstransformator aufweist, welche in einem gemeinsamen Gehäuse angeordnet sind, mit dem ein Tubus verbunden ist, wobei der Hochspannungstransformator zwischen der Röntgenröhre und dem Tubus angeordnet ist und einen Kanal zum Durchtritt eines von der Röntgenröhre ausgehenden Röntgenstrahlenbündels aufweist.

Röntgenstrahler zur Anfertigung von Zahnaufnahmen, deren Tubus dazu dient, aus Gründen des Strahlenschutzes einen gewissen Mindestabstand zwischen dem Fokus der Röntgenröhre und dem Untersuchungsobjekt sicherzustellen, sind meist an einem Wandarm befestigt. Dieser weist eine Anzahl von Gelenken auf, die es gestatten, den Röntgenstrahler in zweckentsprechender Weise auf das Untersuchungsobjekt auszurichten. Um alle erforderlichen Untersuchungen vornehmen zu können, muß der Wandarm erfahrungsgemäß eine solche Länge aufweisen, daß es möglich ist, den horizontal auf die Wand ausgerichteten Röntgenstrahler so zu positionieren, daß die Stirnfläche seines Tubus einen Abstand von ca. 1,5 m zu derjenigen Wand aufweist, an der der Wandarm befestigt ist. Der Wandarm und dessen Gelenke müssen somit nicht unerhebliche Beanspruchungen aufnehmen, so daß geeignete Maßnahmen getroffen werden müssen, um eine ausreichende statische Festigkeit des Wandarmes zu gewährleisten und Schwingungen des Wandarmes, die die Aufnahmequalität mindern würden, zu verhindern. Da der Röntgenstrahler manuell auf das Untersuchungsobjekt ausgerichtet wird, ist außerdem eine handliche Gehäuseform wünschenswert.

Aus der Druckschrift "Heliodent 70" der Siemens AG, Best.-Nr. A19100-M47-A306, ist ein Röntgenstrahler bekannt, der derart aufgebaut ist, daß die Röntgenröhre zwischen dem Hochspannungstransformator und dem Tubus angeordnet ist. Demzufolge weist der bekannte Röntgenstrahler zwar eine schlanke Form auf, die seine Handhabung erleichtert, jedoch ist der Schwerpunkt des bekannten Röntgenstrahlers sehr weit von der Stirnfläche des Tubus entfernt. Dies wirkt sich insbesondere bei Stellungen des Röntgenstrahlers, in denen dieser einen großen Abstand von der Wand aufweist und auf diese ausgerichtet ist, nachteilig auf die Belastung des Tragarmes aus, die um so größer ist, je weiter der Schwerpunkt des Röntgenstrahlers von der an dem Untersuchungsobjekt anliegenden Stirnfläche des Tubus und damit von der Wand entfernt ist.

Ein aus der DE-PS 629 610 bekannter Röntgenstrahler weist einen rohrförmigen Hochspannungstransformator auf, in dessen Bohrung die Röntgenröhre angeordnet ist, deren Röntgenstrahlenbündel durch einen Kanal des Hochspannungstransformators austritt, der quer zu dessen Bohrung verläuft. Im Falle dieses Röntgenstrahlers befindet sich der Hochspannungstransformator somit zumindest teilweise zwischen der Röntgenröhre und dem Tubus, so daß ein verringerter Abstand des Schwerpunktes des Röntgenstrahlers von der Stirnfläche des Tubus vorliegt, was im Hinblick auf die Belastung des Wandarmes vorteilhaft ist. Zur Erzielung dieses Vorteiles ist jedoch ein kompliziert aufgebauter und demzufolge kostspieliger Hochspannungstransformator erforderlich. Außerdem weist der bekannte Röntgenstrahler eine unhandliche Form auf.

Röntgenstrahler der eingangs genannten Art sind jeweils aus der US-PS 4 157 476 und der US-PS 2 137 122 bekannt. Der Schwerpunkt dieser Röntgenstrahler befindet sich infolge der Anordnung des Hochspannungstransformators zwischen der Röntgenröhre und dem Tubus erheblich näher bei der Stirnfläche des Tubus als im Falle von Röntgenstrahlern, bei denen die Röntgenröhre zwischen dem Tubus und dem Hochspannungstransformator angeordnet ist. Infolge des in dem Hochspannungstransformator vorgesehenen Kanals weist dieser im Falle beider bekannter Röntgenstrahler große äußere Abmessungen auf, so daß die bekannten Röntgenstrahler jeweils eine sperrige, etwa quaderförmige Gestalt besitzen, die einer bequemen Handhabung entgegensteht.

Der Erfindung liegt die Aufgabe zugrunde, einen Röntgenstrahler der eingangs genannten Art so auszubilden, daß dessen Schwerpunkt nahe bei der Stirnfläche des Tubus liegt, ohne daß der Röntgenstrahler eine unhandliche Form und der Hochspannungstransformator einen komplizierten Aufbau erhält.

Nach der Erfindung wird diese Aufgabe dadurch gelöst, daß der Hochspannungstransformator eine röhrenförmige Wicklung aufweist, deren Innenwand eine in Richtung der Längssymmetrieachse der Wicklung verlaufende Öffnung begrenzt, durch die der Kanal zum Durchtritt des Röntgenstrahlenbündels verläuft. Es wird deutlich, daß durch die erfindungsgemäße Maßnahme zunächst eine günstige Lage des Schwerpunktes des Röntgenstrahlers, nämlich nahe bei der Stirnfläche des Tubus, sichergestellt ist. Außerdem ist der Hochspannungstransformator infolge des röhrenförmigen Aufbaus seiner Wicklung einfach herstellbar. Darüber hinaus ist es möglich, dem Röntgenstrahler eine schlanke, handliche und insbesondere rotationssymmetrische Form zu geben.

Nach einer Variante der Erfindung ist vorgesehen, daß der Hochspannungstransformator einen rohrförmigen, in der Öffnung der Wicklung angeordneten Kern aufweist, wobei der Kanal zum Durchtritt des Röntgenstrahlenbündels durch die Bohrung des Kernes verläuft. Infolge dieser Anordnung und Ausbildung des Kernes des Hochspannungstransformators ist sichergestellt, daß dieser in radialer Richtung nur einen geringen Bauraum einnimmt und somit einer handlichen Ausbildung des Röntgenstrahlers nicht entgegensteht.

Der erfindungsgemäße Röntgenstrahler kann dann eine besonders handliche Form erhalten, wenn der Hochspannungstransformator im wesentlichen rotationssymmetrisch in bezug auf seine Längsachse ausgebildet ist und diese dem Zentralstrahl des Röntgenstrahlenbündels entspricht.

Nach einer Ausführungsform der Erfindung ist vorgesehen, daß die Wicklung des Hochspannungstransformators eine Primär- und eine Sekundärwicklung enthält, wobei die Sekundärwicklung durch mehrere in Reihe geschaltete und in Richtung der Längsachse des Hochspannungstransformators aufeinanderfolgend angeordnete Wicklungsabschnitte gebildet ist, die die allen Wicklungsabschnitten gemeinsame, rohrförmig ausgebildete Primärwicklung umgeben. Auch diese Maßnahme dient dem einfachen Aufbau des Hochspannungstransformators und stellt infolge der Aufteilung der Sekundärwicklung in einzelne Wicklungsabschnitte eine hohe Spannungsfestigkeit der Sekundärwicklung sicher.

Infolge der beschriebenen Ausbildung des Hochspannungstransformators ist es möglich, Bauteile des Hochspannungsgenerators in raumsparender Weise an der Peripherie des Hochspannungstransformators anzuordnen.

Eine weitere Ausführungsform der Erfindung sieht vor, daß die Röntgenröhre und der Hochspannungsgenerator eine Baueinheit bilden. Durch diese Maßnahme ist es möglich, die Lage der Röntgenröhre relativ zu dem Hochspannungsgenerator bzw. dem in dessen Hochspannungstransformator vorgesehenen Kanal bereits ausserhalb des Gehäuses zu justieren und beide in einem Arbeitsgang gemeinsam in das Gehäuse zu montieren.

Ein Ausführungsbeispiel der Erfindung ist in der beigefügten Zeichnung dargestellt. Es zeigen:

Fig. 1 einen an einem Wandarm angebrachten erfindungsgemäßen Röntgenstrahler, und

Fig. 2 einen erfindungsgemäßen Röntgenstrahler im Längsschnitt.

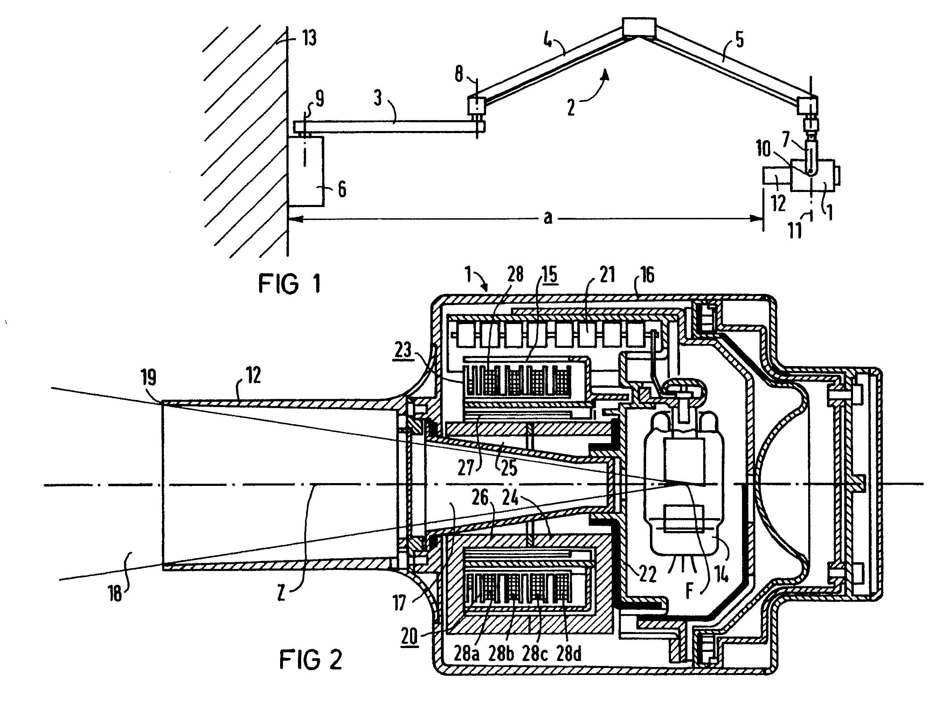

Die Fig. 1 zeigt einen erfindungsgemäßen Röntgenstrahler 1, der an einem insgesamt mit 2 bezeichneten Wandarm angebracht ist. Der Wandarm 2 besteht aus Armabschnitten 3, 4 und 5 sowie einem Wandhalter 6 und einer Halterung 7 für den Röntgenstrahler 1.

Die Halterung 7 ist mit dem Armabschnitt 5, dieser mit dem Armabschnitt 4 und letzterer mit dem Armabschnitt 3 jeweils mittels einer nicht näher dargestellten Gelenkverbindung um eine senkrecht zur Zeichenebene verlaufende Achse schwenkbar verbunden. Der Armabschnitt 4 ist außerdem um die Achse 8 relativ zum Armabschnitt 3 schwenkbar, während dieser um die Achse 9 schwenkbar an dem Wandhalter 6 angebracht ist. Der Röntgenstrahler 1 ist um die Achsen 10 und 11 schwenkbar an der Halterung 7 angeordnet.

Der Röntgenstrahler 1 ist somit innerhalb gewisser Grenzen, die durch die Abmessungen der Armabschnitte 3, 4 und 5 und den Schwenkbereich der Gelenkverbindungen gegeben sind, beliebig positionierbar. In Fig. 1 ist diejenige Position des Röntgenstrahlers 1 dargestellt, in der die Stirnfläche eines an dem Röntgenstrahler 1 angebrachten Tubus 12 den größtmöglichen Abstand a zu der Wand 13 aufweist.

Wie aus Fig. 2 ersichtlich ist, weist der Röntgenstrahler 1 eine Röntgenröhre 14 und einen Hochspannungsgenerator 15 auf, welche in einem gemeinsamen Gehäuse 16 angeordnet sind, mit dem der Tubus 12 verbunden ist.

Der Hochspannungsgenerator 15 weist einen zwischen der Röntgenröhre 14 und dem Tubus 12 angeordneten Hochspannungstransformator 20 mit einem Kanal 17 auf, durch den ein von der Röntgenröhre 14 ausgehendes Röntgenstrahlenbündel 18 verläuft. Es ist ohne weiteres einzusehen, daß der erfindungsgemäße Röntgenstrahler 1 infolge dieser Ausbildung bei einem vorgegebenen Abstand zwischen der Stirnfläche 19 des Tubus 12 und dem Fokus F der Röntgenröhre 14 eine erheblich geringere Baulänge aufweist als ein Röntgenstrahler, bei dem die Röntgenröhre zwischen dem Tubus und dem Hochspannungstransformator angeordnet ist. Außerdem wird unter Heranziehung der Fig. 1 deutlich, daß der erfindungsgemäße Röntgenstrahler 1 bei einem fest vorgegebenen Abstand a zwischen der Wand 13 und der Stirnfläche 19 des Tubus 12 den Wandarm 2 nur wenig belastet, da der Schwerpunkt des erfindungsgemäßen Röntgenstrahlers 1 nahe bei der Stirnfläche 19 des Tubus 12 und damit bei der Wand 13 liegt.

Wie die Fig. 2 weiter zeigt, weist der Hochspannungstransformator 20 eine röhrenförmige Wicklung 23 auf. Infolge ihrer röhrenförmigen Gestalt begrenzt die Wicklung 23 mit ihrer inneren Wand eine Öffnung 25, die sich längs der Achse der Wicklung 23 durch diese hindurch erstreckt. In der Öffnung 25 der Wicklung 23 ist ein im wesentlichen rohrförmiger Kern 24 angeordnet. Wie aus Fig. 2 ersichtlich ist, verläuft der Kanal 17 somit durch die Öffnung 25 der Wicklung 23 bzw. die Bohrung 26 des in der Öffnung 25 angeordneten Kernes 24.

Die Wicklung 23 des Hochspannungstransformators 20 enthält eine Primärwicklung 27 und eine Sekundärwicklung 28, wobei die Sekundärwicklung 28 durch mehrere in Reihe geschaltete und in Richtung der Längsachse des Hochspannungstransformators aufeinanderfolgend angeordnete Wicklungsabschnitte 28a bis 28d gebildet ist, die die allen Wicklungsabschnitten 28a bis 28d gemeinsame, rohrförmig ausgebildete Primärwicklung 27 umgeben.

Der Hochspannungstransformator 20 ist im wesentlichen rotationssymmetrisch ausgebildet und derart angeordnet, daß seine Längsachse dem Zentralstrahl Z des Röntgenstrahlenbündels 18 entspricht.

Infolge der beschriebenen Ausbildung des Hochspannungstransformators 20 und dessen Anordnung relativ zu der Röntgenröhre 14 ist es möglich, dem Gehäuse 16 des Röntgenstrahlers eine schlanke, rotationssymmetrische und damit handliche Gestalt zu geben.

Die übrigen Bauteile des Hochspannungsgenerators 15, z.B. zur Spannungsvervielfachung dienende Kondensatoren 21, sind an der Peripherie des Hochspannungstransformators 20 angeordnet. Die Röntgenröhre 14 und der Hochspannungsgenerator 15 sind mittels eines kappenartigen Bauteiles 22 zu einer Baueinheit zusammengefaßt, die auf einfache Weise in das Gehäuse 16 eingebaut werden kann.

**Patentansprüche**

1. Röntgenstrahler, insbesondere zur Anfertigung von intraoralen Zahnaufnahmen, der eine Röntgenröhre (14) und einen Hochspannungsgenerator (15) mit einem Hochspannungstransformator (20) aufweist, welche in einem gemeinsamen Gehäuse (16) angeordnet sind, mit dem ein Tubus (12) verbunden ist, wobei der Hochspannungstransformator (20) zwischen der Röntgenröhre (14) und dem Tubus (12) angeordnet ist und einen Kanal (17) zum Durchtritt eines von der Röntgenröhre (14) ausgehenden Röntgenstrahlenbündels (18) aufweist, **dadurch gekennzeichnet**, daß der Hochspannungstransformator (20) eine röhrenförmige Wicklung (23) aufweist, deren Innenwand eine in Richtung der Längssymmetrieachse der Wicklung (23) verlaufende Öffnung (25) begrenzt, durch die der Kanal (17) zum Durchtritt des Röntgenstrahlenbündels (18) verläuft.

2. Röntgenstrahler nach Anspruch 1, **dadurch gekennzeichnet**, daß der Hochspannungstransformator (20) einen rohrförmigen, in der Öffnung (25) der Wicklung (23) angeordneten Kern (24) aufweist, wobei der Kanal (17) zum Durchtritt des Röntgenstrahlenbündels (18) durch die Bohrung (26) des Kernes (24) verläuft.

3. Röntgenstrahler nach Anspruch 1 oder 2, **dadurch gekennzeichnet**, daß der Hochspannungstransformator (20) im wesentlichen rotationssymmetrisch in bezug auf seine Längsachse ausgebildet ist, die dem Zentralstrahl (Z) des Röntgenstrahlenbündels (18) entspricht.

4. Röntgenstrahler nach einem der Ansprüche 1 bis 3, **dadurch gekennzeichnet**, daß die Wicklung (23) des Hochspannungstransformators (20) eine Primär- und eine Sekundärwicklung (27 bzw. 28) enthält, wobei die Sekundärwicklung (28) durch mehrere in Reihe geschaltete und in Richtung der Längsachse des Hochspannungstransformators (20) aufeinanderfolgend angeordnete Wicklungsabschnitte (28a, 28b, 28c, 28d) gebildet ist, die die allen Wicklungsabschnitten (28a, 28b, 28c, 28d) gemeinsame, rohrförmig ausgebildete Primärwicklung (27) umgeben.

5. Röntgenstrahler nach einem der Ansprüche 1 bis 4, **dadurch gekennzeichnet**, daß Bauteile (21) des Hochspannungsgenerators (15) an der Peripherie des Hochspannungstransformators (20) angeordnet sind.

6. Röntgenstrahler nach einem der Ansprüche 1 bis 5, **dadurch gekennzeichnet**, daß die Röntgenröhre (14) und der Hochspannungsgenerator (15) eine Baueinheit bilden.

**Claims**

1. An x-ray source, in particular for preparing intraoral tooth exposures, which has an x-ray tube (14) and a high-voltage generator (15) with a high-voltage transformer (20), which are arranged in a common housing (16), to which a barrel (12) is connected, wherein the high-voltage transformer (20) is arranged between the x-ray tube (14) and the barrel (12), and has a channel (17) for the passage of an x-ray beam (18), emanating from the x-ray tube (14), characterised in that the high-voltage transformer (20) has a tubular winding (23), the inner wall of which defines an opening (25), extending in the direction of the longitudinal symmetrical axis of the winding (23), through which extends the channel (17) for the passage of the x-ray beam (18).

2. An x-ray source according to claim 1, characterised in that the high-voltage transformer (20) has arranged in the opening (25) of the winding (23) a core (24), wherein the channel (17) for the passage of the x-ray beam (18) extends through the bore (26) of the core (24).

3. An x-ray source according to claim 1 or 2, characterised in that the high-voltage transformer (20) is constructed with substantially rotational symmetry relative to its longitudinal axis, which corresponds with the central ray (Z) of the x-ray beam (18).

4. An x-ray source according to one of claims 1 to 3, characterised in that the winding (23) of the high-voltage transformer (20) comprises a primary and a secondary winding (27 or 28), wherein the secondary winding (28) is formed by several winding sections (28a, 28b, 28c, 28d), connected in series and one on top of the other in the direction of the longitudinal axis of the high-voltage transformer (20), which sections surround the tubular-formed primary winding (27), common to all winding sections (28a, 28b, 28c, 28d).

5. An x-ray source according to one of claims 1 to 4, characterised in that the components (21) of the high-voltage generator (15) are arranged on the periphery of the high-voltage transformer (20).

6. An x-ray source according to one of claims 1 to 5, characterised in that the x-ray tube (14) and the high-voltage generator (15) form one component.

**Revendications**

1. Émetteur de rayons x, notamment pour la réalisation de radiographies dentaires intra-orales, comportant un tube à rayons X (14) et un générateur de haute tension (15) équipé d'un transformateur à haute tension (20), qui sont disposés dans un carter commun (16) auquel est raccordé un localisateur (12), et dans lequel le transformateur de haute tension (20) est disposé entre le tube à rayons (14) et le localisateur (12) et comporte un canal (17) pour 1e passage d'un faisceau de rayons X (18) sortant du tube à rayons x (14), caractérisé par le fait que le transformateur à haute tension (20) possède un enroulement tubulaire (23), dont la paroi intérieure délimite une ouverture (25), qui s'étend dans la direction de l'axe longitudinal de symétrie d'enroulement (23) et que traverse le canal (17) pour le passage du faisceau de rayons X (18).

2. Émetteur à rayons x suivant la revendication 1, caractérisé par le fait que le transformateur à haute tension (20) possède un noyau tubulaire (24) disposé dans l'ouverture (25) de l'enroulement (23), le canal (17) pour le passage du faisceau de rayons X (18) s'étendant dans le perçage (26) du noyau (24).

3. Émetteur de rayons x suivant la revendication 1 ou 2, caractérisé par le fait que le transformateur à haute tension (20) possède essentiellement une symétrie de révolution par rapport à son axe longitudinal, qui correspond au rayon central (Z) du faisceau de rayons X (18).

4. Émetteur de rayons X suivant les revendications 1 à 3, caractérisé par le fait que l'enroulement (23) du transformateur à haute tension (20) contient un enroulement primaire et un enroulement secondaire (27 ou 28), l'enroulement secondaire (28) étant formé par plusieurs sections d'enroulement (28a,28b,28c,28d), qui sont branchées en série et disposées à la suite les unes des autres dans la direction de l'axe longitudinal du transformateur à haute tension (20) et qui entourent l'enroulement primaire (27) de forme tubulaire, commun à toutes les sections d'enroulement (28a,28b,28c,28d).

5. Émetteur de rayons x suivant l'une des revendications 1 à 4, caractérisé par le fait que des composants (21) du générateur à haute tension (15) sont disposés sur le pourtour du transformateur à haute tension (20).

6. Émetteur de rayons X suivant l'une des revendications 1 à 5, caractérisé par le fait que le tube à rayons X (14) et le générateur à haute tension (15) forment une unité de construction.

FIG 1

FIG 2